# EUROPEAN PATENT SPECIFICATION

(11) **EP 2 465 564 B1**
(45) Date of publication and mention of the grant of the patent: **08.02.2017**
(21) Application number: 10195021.0
(22) Date of filing: 14.12.2010
(51) Int. Cl.: A61M 16/06, A61M 16/08, A61M 39/10

(54) **Facial mask for treating sleep disorders with locking means for blocking the rotatable gas connector**
Gesichtsmaske zur Behandlung von Schlafstörungen mit Verriegelungsvorrichtungen zum Arretieren des drehbaren Gasanschlusses
Masque facial permettant de traiter les troubles du sommeil avec des supports de verrouillage pour bloquer le connecteur de gaz rotatif

(43) Date of publication of application: 20.06.2012
(73) Proprietor: Air Liquide Medical Systems, 92160 Antony (FR)
(72) Inventor: Alberici, Luca, 25128 Brescia (IT); Rivetti, Alberto, Rodengo Saiano (BS) 25050, Rovato (BS) (IT); Sandoni, Giuseppe, 25124 Brescia (IT); Lopez, Giullaume, 22560 Pleumeur Bodou (FR)
(74) Representative: Pittis, Olivier

(56) References cited:
- US-A1- 2003 094 177
- US-A1- 2005 020 972
- US-A1- 2008 276 937

## Description

The invention concerns a respiratory mask, in particular a facial mask, for use in the treatment of respiratory conditions or diseases, such as obstructive sleep apnea. The respiratory mask comprises a rotating elbow-connector that is able to rotate with respect to the mask body.

Masks, such as facial or nasal masks, are commonly used for delivering non-invasive positive pressure ventilation (NPPV) or for nasal continuous positive airway pressure (N-CPAP) therapy in sleep disordered breathing (SDB) conditions, such as obstructive sleep apnea (OSA). They typically deliver a flow of breathable gas for, or to assist in, patient respiration, especially during the night, i.e., when the patient is sleeping.

Such a mask assembly typically comprises a rigid or semi-rigid hollow shell, also called a mask body, defining a breathing chamber that receives at least a part of the patient's nose and/or mouth, and further comprising a soft face-contacting cushion that comes into contact with the patient's face, and a forehead support that is either pivotable or not pivotable and a headgear for correctly positioning, maintaining and/or securing the mask on the head of a patient. The shell of the mask or mask body is connected to a gas supply line which delivers a respiratory gas, such as air under pressure, into the breathing chamber of the shell through a gas opening or inlet orifice arranged in the wall of the mask body. The connection between the gas line and the mask body is usually obtained by means of a hollow connector comprising an internal passage for the gas. The connector can have an elbow-shape, i.e. be curved, or any other shape.

A rotatable connector that rotates around the axis of the inlet orifice is known to be used to correctly position the gas line, which is typically a flexible conduit, with respect to the mask.

The problem that exists with such rotatable connectors, is that they can rotate while the patient is sleeping, thereby causing a bad gas seal of the mask on the patient's face and thus leading to gas leaks. While attempts for modifying the existing connectors have been made, so far the resulting masks are not totally satisfying.

Further, US-A-2003/0094177 discloses a mask for supplying gases to a patient comprising a L-shaped swiveled connector that is capable of being fixed in a first position where the connector is freely rotatable within the mask and a second position wherein the connector is blocked by locking means and does not rotate. The locking means comprises ridges and protrusions cooperating together.

Hence, the problem to be solved is to provide an improved mask, especially a facial mask, for allowing an easy and efficient angular positioning of the tubing elements, i.e. the flexible conduit, and of the connector with respect to the mask body so as to limit the risks of creating gas leaks while the patient is sleeping. The solution that the present invention provides is a respiratory mask comprising a mask body with an internal chamber and a gas inlet orifice, said gas inlet orifice having an axis (AA), said gas inlet orifice being in fluid communication with said internal chamber, and a hollow connector with an internal gas passage being connected to said gas inlet orifice so that the internal passage of the hollow connector is in fluid communication with said internal chamber, said hollow connector being rotatable around the axis (AA), wherein:
- the hollow connector is further mobile in translation along the axis (AA) between at least a first axial position, wherein the connector can freely rotate around said axis (AA), and a second axial position, wherein the connector is locked in a fixed position and can not rotate around said axis (AA),
- and the mask further comprises locking means for locking and for maintaining the hollow connector blocked in said second axial position, said locking means comprising lips including an inner lip carried by the mask body and external lips carried by the hollow connector, wherein the locking means further comprise at least one notch carried by the mask body, and at least one abutment carried by the hollow connector, said at least one abutment at least partially penetrating into said notch for blocking the rotation of the hollow connector around axis, when the hollow connector is in the second axial position.

The mask according to the present invention can further comprise one or more of the features of the dependent claims.

One embodiment of a facial mask according to the present invention is shown in the enclosed Figures, among which :
- Figure 1 represents a facial mask according to the present invention,
- Figures 2-8 show an embodiment of locking means and connector of a facial mask of the present invention, and
- Figures 9-15 represent an embodiment of locking means and connector of a facial mask of the present invention.

As illustrated in the Figures, a respiratory facial mask according to the present invention comprises a rigid or semi-rigid hollow shell or mask body 1 defining an internal breathing chamber 2 or volume, wherein respiratory gas, such as air under pressure, is introduced via an inlet port 3 which is connected a gas feeding line, such as a flexible gas conduit, by means of a tubular hollow connector 4. Preferably, the connector 4 has a general elbow-shape and comprises an internal passage for the gas.

The gas inlet orifice 3 is arranged at the center of the mask body 1 and through the wall of the mask thereby allowing air under pressure to be introduced in the breathing chamber 2 that receives the nose and mouth of the patient.

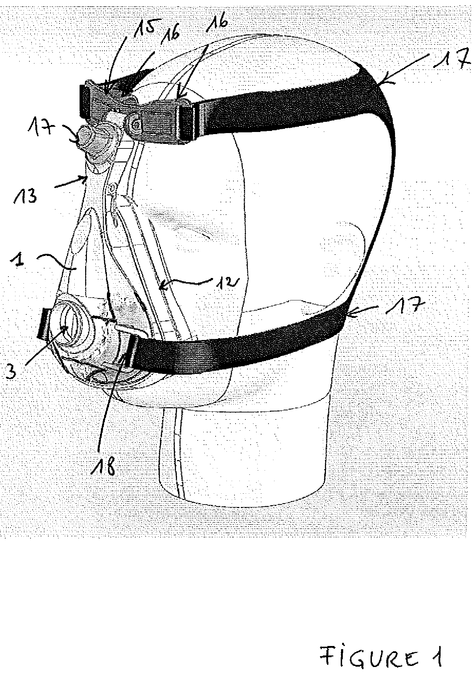

The mask body 1 is preferably made of a polymer material, such as polycarbonate (PC), polypropylene (PP), ABS, nylon or polystyrene (PS), and is configured so as to be able to receive at least a part of the patient's nose. In other words, the patient introduces his/her nose and mouth into the internal volume of the breathing chamber 2 of the mask body 1 and breathes the pressurized gas contained therein. The mask body 1 preferably has a general triangular or quasi-triangular three-dimensional shape as shown in Figure 1, for example.

The mask body 1 further comprises an expansion part 13 arranged on the top of the mask body 1 and projecting upwardly from said mask body 1, i.e. projecting away from the external surface of the mask body 1. The expansion part 13 comprises a traversing orifice 14 that is preferably located at the distal end 13a of the expansion part 13. Preferably, the mask body 1 and the expansion part 13 are made of polymeric material that is molded in one piece so as to obtain an expansion part 13 that is integral with the rest of the mask body 1.

Further, as represented in Figure 1, a forehead support 15 is connected to the mask body 1 by means of a pivotable holding arm fixed to or carried by either the mask body 1 or the expansion part 13, preferably the expansion part 13. The forehead support 15 can comprise one or several pillows 16 of soft and comfortable material that come into contact with the patient's forehead.

An acting piece (not visible in Fig. 1) is arranged in the traversing orifice 14 of the expansion part 13 and is mobile, preferably in translation, in said traversing orifice 13 so as to cooperate with the holding arm for pivoting said holding arm when said acting piece moves in the traversing orifice 14 of the expansion part 13. Further, the forehead support 15 can also pivot with respect to the holding arm 13. Actually, the back or forward motion of the acting piece in the traversing orifice 14 is obtained by manual rotation by the user of a rotating knob 17 cooperating with the acting piece--when said rotating knob 17 is manually operated, i.e. turned clockwise or counterclockwise, by the user. The maximum course of the knob 6 is about 360° or less.

Furthermore, in order to ensure a tight positioning of the nasal mask on the patient's face and to increase the comfort for the patient, the peripheral border or edge 11 of the mask body 1 comprises a cushion 12 made of soft, resilient, elastomeric material that comes into contact with the patient's face. Said cushion 12 has a central aperture for receiving at least a part of the patient's nose. More precisely, the border 11 and the cushion 12 have a general triangular or saddle-shape structure so as to match the contours of the nasal region including the upper nasal bridge region, the cheek regions on both sides of the nose, and the lower chin region of the patient. The cushion 12 can comprise one or several membranes, preferably two membranes. Such cushion 12 and mask body 1 structures are well-known in the art and taught by many documents, such as EP-A-1334742, EP-A-264772, EP-A-956069, EP-A-874667, US-A-2,931,356 or EP-A-1479406.

Furthermore, a headgear 17 comprising straps can be connected to the mask body 1, by fixing means 18 such as hooks and slots cooperating together, for fixing the straps of the headgear and thereby maintaining the mask in the selected position on the head of the patient during the use of the mask and thus obtain an efficient treatment of sleep disorders, such as OSA or similar disorders. The headgear-fixing means 18 can be made integral with the shell 1 body. Such structures are well known in the art and disclosed in many documents, such as, for example, EP-A-1334742, EP-A-462701, EP-A-1985327 or EP-A-956069.

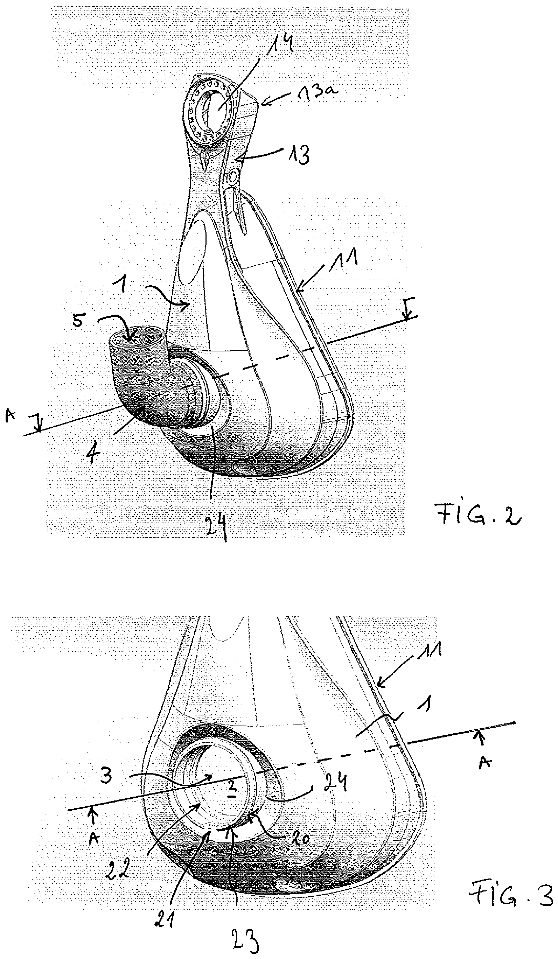

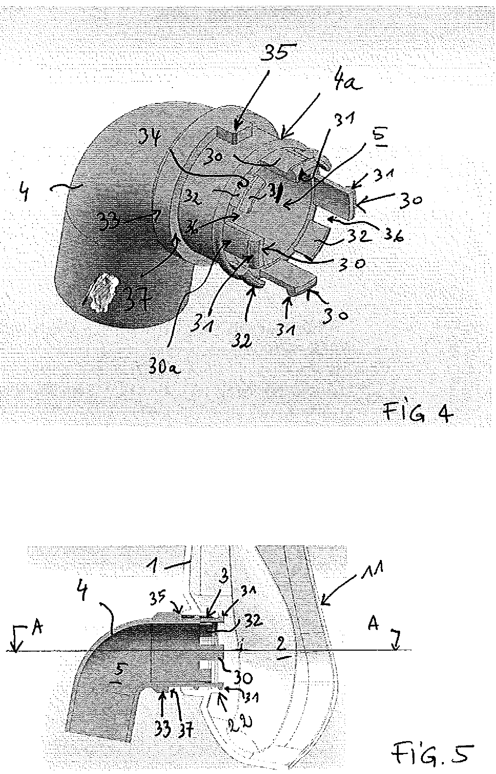

The shell or mask body 1 is fluidly linked to a gas supply line, such as a flexible hose or conduit conveying respiratory gas to the mask, by means of a tubular hollow connector 4 which delivers the respiratory gas, such as air under pressure, into the breathing chamber 2 of the shell 1 through a gas inlet 3 arranged in the mask body 1 as represented in Figures 2, 3, 5 and 8. More precisely, the tubular connector 4, that can be made of polymer material, has a circular section and preferably an elbow or curved form as shown in Figure 4.

According to the present invention, the hollow connector 4 is rotatable, i.e. can freely turns around the axis AA of the inlet orifice 3, as shown in Figures 2, 3 and 5, or can be locked, i.e. blocked, in a desired fixed angular position so that the rotation of the hollow connector is not possible around the axis AA. Actually, the hollow connector 4 is rotatable around axis AA as well as moveable in translation on axis AA, i.e. back and forward vis-a-vis the mask body 1, between at least a first position wherein the hollow connector can freely rotate around said axis AA and at least a second position where no rotation is allowed, i.e. the connector 4 is blocked in rotation. In other words, for locking the connector 4 in a given or desired angular position around axis AA, the user should first put the connector 4 in the first axial position of the connector 4 (if the connector 4 is not already in that first axial position), then rotate the connector 4 to get the desired angular position and then exert a manual pressure on the connector 4 so as the connector 4 translates forward on axis AA in the direction of the mask body 1 to its second axial position. Hence, the connector 4 is locked in said second axial position and thereby has been locked into its angular position. Locking means or a locking system are used for locking and maintaining the hollow connector 4 in said second axial position so as to prohibit any free rotation of the connector around axis AA.

An of possible locking means is shown in Figures 2-8. Said locking means comprises particular structures cooperating together which are carried, on the one hand, by the inlet orifice 3 and, on the other hand, by the hollow connector 4. More precisely, in this first embodiment, as shown in Fig. 3, the inlet orifice 3 has a three-dimensional shape as the inlet orifice 3 is formed by a tubular element 20 of axis AA, projecting outwardly of the external surface of the mask body 1. Further, said tubular element 20 is preferably made integral with the mask body 1, for instance by molding in one piece. Furthermore, said tubular element 20 comprises one or several cuts or the like made in the outer peripheral border 23 of the tubular element 20 so as to form one or several notches 21. The internal cylindrical surface of the tubular element 20 carries an inner lip 22 having a semi-cylindrical shape that is used for retaining the connector 4 in its first position as detailed below.

The hollow connector 4 also has a particular structure that is complementary to the structure of the inlet orifice 3 so that both structures can cooperate together. More precisely, as shown in Fig. 4, the proximal end 4a of the connector 4 that cooperates with the inlet orifice 3 comprises several long fingers or strips 30 projecting outwardly with respect to the peripheral border or edge 34 of the proximal end 4a of the connector 4. Further, the proximal end 4a of the connector 4 also comprises several short fingers or strips 32 that also project outwardly with respect to the peripheral border or edge 34 of the proximal end 4a of the connector 4. Each of said long and short fingers or strips 30, 32 is fixed by its distal end to the proximal end 4a of the hollow connector 4 and further carries an external lip 31 on its outer surface forming, at its distal end, a small abutment radially-oriented toward the exterior of the hollow connector 4. The short fingers or strips 32 have a length that is shorter than the one of the long fingers 30, for instance the length of the short fingers is from about 3 to 6 mm, whereas the length of the long fingers 30 is from about 6 to 12 mm. Preferably, the connector 4 comprises between 2 and 20 long and short fingers 30, 32, typically about 4 of each.

More preferably, the long and short fingers 30, 32 are arranged in an alternate way as represented in Figure 4, i.e. each long finger 30 is positioned between 2 short fingers, and reciprocally, while being spaced by free spaces 36 like the crenels of a castle.

Further, the hollow connector 4 also carries on its peripheral external surface one (or several) abutment 35 and preferably a peripheral ring 33 comprising a front edge 37 that comes into contact with the outer peripheral border 23 of the tubular element 20 when the connector 4 is in its second position as explained below. The abutment 35 is dimensioned so as to have a form and size that matches the form of the notch(es) 21 of the tubular element 20.

The short fingers 32, long fingers 30, ring 33 and abutment 35 are preferably made in one-piece by molding with the rest of the connector 4. Preferably, the short fingers 32 and the long fingers 30 are made of a resilient material that is flexible or at least slightly deformable, such as a polymer material, e.g. polycarbonate (PC), polypropylene (PP), ABS, nylon or polystyrene (PS), or similar material.

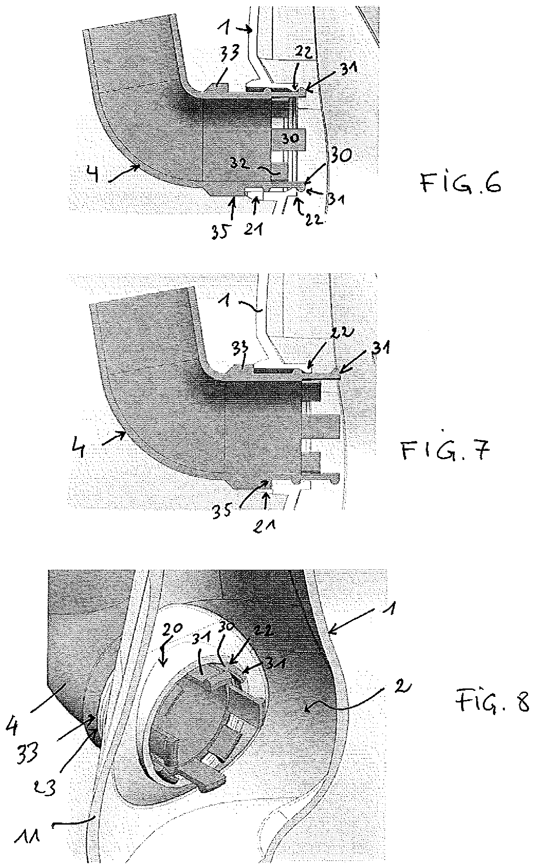

The outer diameter of the proximal end 4a of the hollow connector 4 has a shape and size that matches the inner wall of the tubular element 20, for instance an outer diameter of between about 10 and 50 mm. When the proximal end 4a of the hollow connector 4 is inserted into the tubular element 20 so that it is in its first axial position along the axis AA, it is retained therein by the lips 31 of the long fingers 30 that come in abutment against the inner lip 22 of the tubular element 20 as shown in Figures 5 and 6.

In the first position, the connector 4 can freely rotate around the axis AA so as to occupy a desired angular position around said axis AA. For instance, Figures 5 and 6 illustrate the connector 4 oriented in two different angular positions spaced 180° around the axis AA for a connector 4 in its first position along the axis AA. In the first axial position, the abutment 35 does not engage, i.e. penetrate into, the notch 21 as shown in Fig. 6.

Once the connector 4 has been placed in the desired angular position of Figure 6, by motion around the axis AA, i.e. by rotation, the user can exert on said connector 4 a manual pressure so as to get a translation of the connector 4 along the axis AA thereby reaching its second axial position along the axis AA. Actually, operating such a translation of the connector 4 along the axis AA involves a translation motion of the connector 4 axially in the direction of the mask body 1. This involves a motion of the short fingers 32 in the direction of the inner lip 22 of the tubular element 20 as shown in Figure 7 and further the abutment 35 penetrates into the notch 21 of the tubular element 20. The external lip 31 of each of the short fingers 32 comes first into contact with the inner lip 22 of the tubular element 20 and then, as short fingers 32 are made of resilient material, the external lips 31 of the short fingers 32 pass above said inner lip 22 and come in abutment with the other side of said inner lip 22, as shown in Figure 8, so as to retain the connector 4 blocked and locked in the second axial position. Meanwhile, the abutment 35 that is lodged into the notch 21 of the tubular element 20 avoids or prohibits any angular motion, i.e. rotation of the connector 4 with respect to the mask shell 1. Further, the course of the connector 4 in the direction of the mask body 1 is limited by the annular ring 33 that comes in abutment with the outer peripheral border 23 of the tubular element 20 as represented in Figure 8.

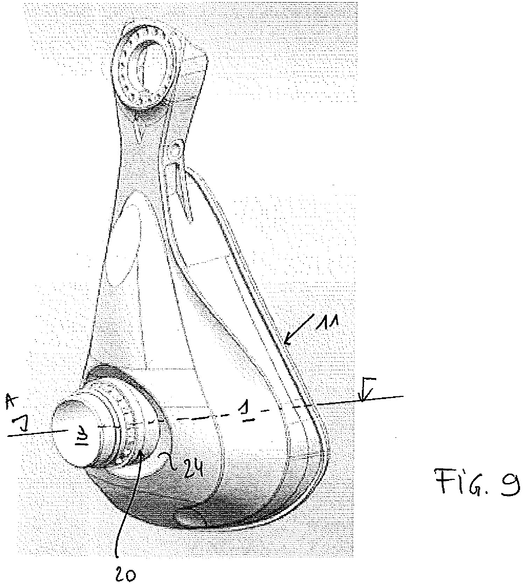

An example of another locking means is shown in Figures 9-15. Here again, the locking means comprises particular structures cooperating together which are carried, on the one hand, by the inlet orifice 3 and, on the other hand, by the hollow connector 4. More precisely, in this example as shown in Figure 9, the inlet orifice 3 has a three-dimensional shape as it is formed, here again, by a tubular element 20 of axis AA, projecting outwardly of the external surface of the mask body 1, which tubular element 20 is preferably arranged in a lodging 24 in recess formed in the outer surface of the mask. Said tubular element 20 is preferably made integral with the mask body 1, for instance, by molding in one piece.

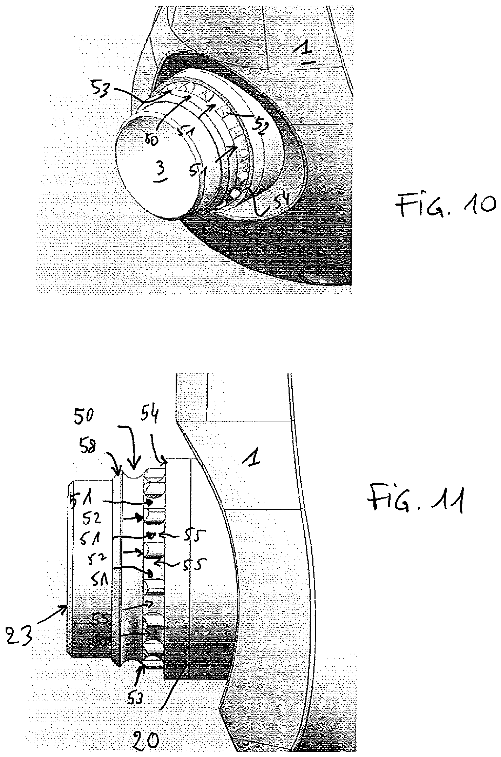

In this example as detailed in Figures 10-11, the tubular element 20 comprises two successive peripheral grooves 50, 51, i.e. annular recesses, made in the outer peripheral wall of the tubular element 20, said grooves 50, 51 having roughly the same shape. The first groove 50 is located between the outer peripheral border 23 of the tubular element 20 and the second groove 51, whereas the second groove 51 is arranged between the first groove 50 and a shoulder 54 situated around the peripheral external surface of the tubular element 20, i.e. close to the body 1 of the mask.

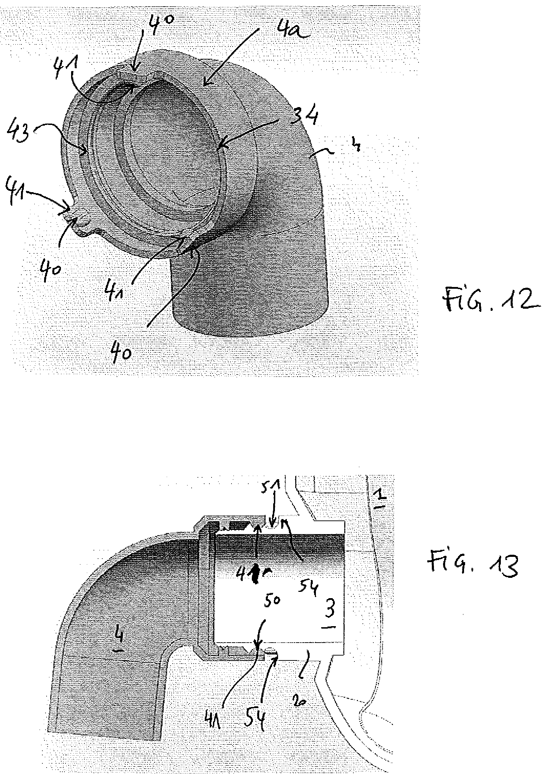

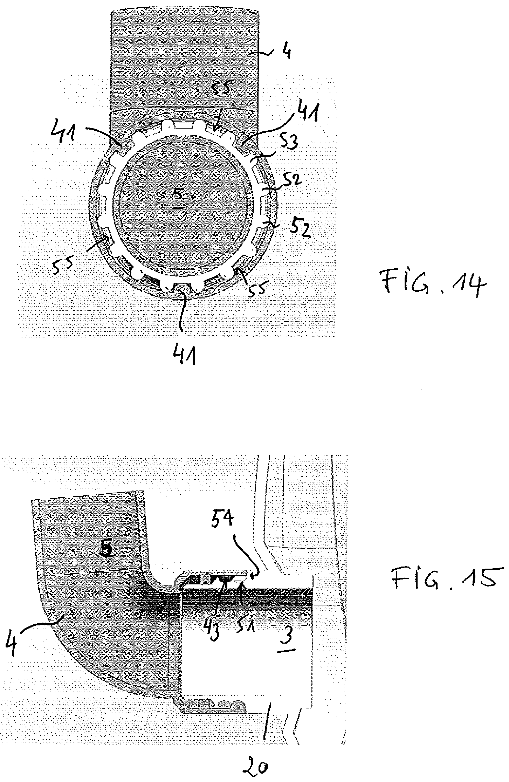

In the second groove 51 are arranged a plurality of little wall portions 52, e.g. 16 wall portions, that are radially and outwardly projecting so as to divide the second groove 51 in a plurality of little lodgings 55, e.g. 16 lodgings, each formed by a portion of the second groove 51 and delimited said wall portions 52, as illustrated on Figures 11 and 14. The little wall portions 52 are angularly distributed, in a regular manner, all along the annular second groove 51. Further, the hollow connector 4 has also in this case a particular structure that fits the structure of the inlet orifice 3 so that both structures can cooperate together. More precisely, the internal cylindrical surface of the hollow connector 4 carries an inner annular lip 43 having a semi-cylindrical shape that is used for retaining the connector 4 in its first and second axial position along the axis AA, and for ensuring that no air is lost between the connector 4 and the inlet orifice 3, as shown in Figures 12, 13 and 15.

Furthermore, as illustrated in Figure 12, the proximal end 4a of the connector 4 that cooperates with the inlet orifice 3 comprises several fingers or strips 40, e.g. 3 fingers 40, projecting outwardly with respect to the peripheral border or edge 34 of the proximal end 4a of the connector 4. Each of said fingers or strips 40 is fixed by its distal end to the proximal end 4a of the hollow connector 4 and comprises at the proximal end of the fingers or strips 40 a nipple 41 having a shape that fits with the shape of the little lodgings 55 formed in the second groove 51. Preferably, the connector 4 comprises between 2 and 20 fingers 40, typically between 2 and 10. The fingers 40 and nipples 41 are preferably made in one-piece by molding with the rest of the connector 4. Hence, they are advantageously made of a resilient material that is flexible or at least slightly deformable, such as a polymer material, e.g. polycarbonate (PC), polypropylene (PP), ABS, nylon or polystyrene (PS) or similar.

The outer diameter of the proximal end 4a of the hollow connector 4 has a shape and size that matches the inner wall of the tubular element 20, for instance, an outer diameter of between about 10 and 50 mm. When the proximal end 4a of the hollow connector 4 is connected to the tubular element 20 so that it is in the first axial position along the axis AA, the proximal end 4a is retained therein by the nipples 41 that are lodged in the first groove 50 of the tubular element 20, as shown in Figure 13.

In the first axial position, the connector 4 can freely rotate around the axis AA so as to occupy a desired angular position around said axis AA. For instance, Figures 13 and 15 illustrate the connector 4 oriented in two different angular positions spaced 180° around the axis AA for a connector 4 in its first position along the axis AA. In the first axial position, the nipples 41 do not engage, i.e. penetrate into, the lodgings 51 of the second groove 51 as shown in Figure 13.

Once the connector 4 has been placed in the desired angular position of Figure 15, by rotation around the axis AA, the user can exert on said connector 4 a manual pressure so as to get a translation of the connector 4 along the axis AA, in the direction of the mask body 1, thereby reaching its second axial position along the axis AA. Actually, operating such a translation of the connector 4 along the axis AA involves a translation motion of the connector 4 axially in the direction of the mask body 1. This involves a motion of the fingers 40 carrying the nipples 41, in the direction of the second groove 51 of the tubular element 20 and further their penetration into the lodgings 55 formed in the second groove 51. The nipples 41 are then retained in said lodgings 55, thanks to the wall 53 separating the first and the second grooves 50, 51 and the course in translation toward the mask body 1, is blocked by the annular shoulder 54 of the tubular element 3 against which abuts the peripheral border or edge 34 of the proximal end 4a of the connector 4. The connector 4 is thus in its second axial position and is blocked in rotation and locked in translation in said second axial position thanks to the cooperation of the nipples 41 with the lodgings 55 of the tubular element 3. This avoids any angular motion, i.e. rotation of the connector 4 with respect to the mask shell 1.

A particular feature of this example of the mask is that, depending on the height or size chosen for the wall portions 52, when it is in the second position along the axis AA, the connector 4 can be either totally blocked (i.e., important size/height of the wall portions 52) or still be rotatable around the axis AA (i.e., medium size/height of the wall portions 52), when exerting a rotation force on said connector. In the latter case, the connector can not however rotate freely as its rotation requires a minimum rotation force exerted by the user and applied on the connector, i.e., a voluntary rotational motion around the axis AA.

The facial mask of the present invention can be used in a method for treatment of a respiratory disorder or condition, for example, in non-invasive positive pressure ventilation (NPPV) or in a continuous positive airway pressure (CPAP) therapy of sleep disordered breathing (SDB) conditions, such as, for example, obstructive sleep apnea (OSA).

## Claims

1. Respiratory mask comprising a mask body (1) with an internal chamber (2) and a gas inlet orifice (3), said gas inlet orifice (3) having an axis (AA), said gas inlet orifice (3) being in fluid communication with said internal chamber (2), and a hollow connector (4) with an internal gas passage (5) being connected to said gas inlet orifice (3) so that the internal passage (5) of the hollow connector (4) is in fluid communication with said internal chamber (2), said hollow connector (4) being rotatable around the axis (AA), wherein :
- the hollow connector (4) is further mobile in translation along the axis (AA) between at least : a first axial position, wherein the connector can freely rotate around said axis (AA) and a second axial position, wherein the connector is locked in a fixed position and cannot freely rotate around said axis (AA),
- and the mask further comprises locking means for locking and for maintaining the hollow connector (4) blocked in said second axial position, said locking means comprising lips (22, 31) including an inner lip (22) carried by the mask body (1) and external lips (31) carried by the hollow connector (4),
**characterized in that** the locking means further comprise at least one notch (21) carried by the mask body (1), and at least one abutment (35) carried by the hollow connector (4), said at least one abutment (35) at least partially penetrating into said notch (21) for blocking the rotation of the hollow connector around axis (AA), when the hollow connector (4) is in the second axial position.

2. Respiratory mask according to Claim 1, **characterized in that** the hollow connector (4) has an elbow-shape.

3. Respiratory mask according to any one of the preceding Claims, **characterized in that** the inner lip (22) and the at least one notch (21) are arranged on a tubular element (20) carried by the mask body (1), the gas inlet orifice (3) traversing said tubular element (20).

4. Respiratory mask according to Claim 3, **characterized in that** the tubular element (20) projects outwardly of the external surface of the mask body (1).

5. Respiratory mask according to any one of the preceding Claims, **characterized in that** the tubular element (20) is made integral with the mask body (1).

6. Respiratory mask according to any one of Claims 4 and 5, **characterized in that** the tubular element (20) comprises at least one cut made in the outer peripheral border (23) of said tubular element (20) so as to form said at least one notch (21).

7. Respiratory mask according to any one of Claims 4 and 5, **characterized in that** the tubular element (20) has an internal cylindrical surface carrying the inner lip (22).

8. Respiratory mask according to any one of the preceding Claims, **characterized in that** several lips (31) are carried by long and short fingers (30, 32) arranged on the hollow connector (4), the short fingers (32) having a length that is shorter than the one of the long fingers (30).

9. Respiratory mask according to any one of the preceding Claims, **characterized in that** the long and short fingers (30, 32) arranged on the hollow connector (4) project outwardly with respect to the peripheral edge (34) of the proximal end (4a) of the connector (4), each of said long and short fingers (30, 32) being fixed by its distal end to the proximal end (4a) of the hollow connector (4) and further carries an external lip (31) on its outer surface forming, at its distal end, a small abutment radially-oriented toward the exterior of the hollow connector (4).

10. Respiratory mask according to any one of the preceding Claims, **characterized in that** the connector (4) comprises between 2 and 20 long and short fingers (30, 32), the length of the short fingers (32) being from 3 to 6 mm and the length of the long fingers (30) being from 6 to 12 mm.

11. Respiratory mask according to any one of the preceding Claims, **characterized in that**, when the proximal end (4a) of the hollow connector (4) is inserted into the tubular element (20), said connector (4) is retained:
- in the first axial position, by the external lips (31) of the long fingers (30) that come in abutment against the inner lip (22) of the tubular element (20), and
- locked in the second axial position, by the external lips (31) of the short fingers (32) coming in abutment with said inner lip (22).

12. Respiratory mask according to any one of the preceding Claims, **characterized in that** the abutment (35) is dimensioned so as to have a form and size that match the form of the at least one notch (21) of the tubular element (20).

13. Respiratory mask according to any one of the preceding Claims, **characterized in that** the respiratory mask is a facial mask.

14. Respiratory mask according to any one of the previous claims, **characterized in that** the internal chamber (2) comprises a peripheral border (11) and a cushion (12) being fixed to said peripheral border (11), said cushion (12) having a central aperture for receiving at least part of the patient's nose and/or mouth.

15. Respiratory mask according to any one of the preceding Claims, **characterized in that** the respiratory mask further comprises a forehead support (9), a headgear and fixing means (19, 20) for fixing the headgear to the mask body (1).

## Patentansprüche

1. Beatmungsmaske, die einen Maskenkörper (1) mit einer Innenkammer (2) und einer Gaseinlassöffnung (3) umfasst, wobei die Gaseinlassöffnung (3) eine Achse (AA) hat, wobei die Gaseinlassöffnung (3) in fluidtechnischer Verbindung mit der Innenkammer (2) steht, und einen hohlen Steckverbinder (4) mit einer inneren Gaspassage (5), die mit der Gaseinlassöffnung (3) verbunden ist, so dass die innere Passage (5) des hohlen Steckverbinders (4) in fluidtechnischer Verbindung mit der Innenkammer (2) steht, wobei der hohle Steckverbinder (4) um die Achse (AA) drehbar ist, wobei:
- der hohle Steckverbinder (4) ferner in Verschiebung entlang der Achse (AA) zwischen mindestens Folgendem beweglich ist: einer ersten axialen Position, in der der Steckverbinder frei um die Achse (AA) drehen kann, und einer zweiten axialen Position, in der der Steckverbinder in einer stationären Position verriegelt ist und nicht frei um die Achse (AA) drehen kann,
- und die Maske ferner Verriegelungsmittel umfasst, um den hohlen Steckverbinder (4) in der zweiten axialen Position zu verriegeln und blockiert zu halten, wobei die Verriegelungsmittel Lippen (22, 31) umfassen, die eine Innenlippe (22), die von dem Maskenkörper (1) getragen wird, und Außenlippen (31), die von dem hohlen Steckverbinder (4) getragen werden, umfasst,
**dadurch gekennzeichnet, dass** die Verriegelungsmittel ferner mindestens eine Kerbe (21) umfassen, die von dem Maskenkörper (1) getragen wird, und mindestens einen Anschlag (35), der von dem hohlen Steckverbinder (4) getragen wird, wobei der mindestens eine Anschlag (35) mindestens teilweise in die Kerbe (21) eindringt, um die Drehung des hohlen Steckverbinders um die Achse (AA) zu blockieren, wenn der hohle Steckverbinder (4) in der zweiten axialen Position ist.

2. Beatmungsmaske nach Anspruch 1, **dadurch gekennzeichnet, dass** der hohle Steckverbinder (4) eine Ellbogenform hat.

3. Beatmungsmaske nach einem der vorhergehenden Ansprüche, **dadurch gekennzeichnet, dass** die Innenlippe (22) und die mindestens eine Kerbe (21) auf einem röhrenförmigen Element (20), das von dem Maskenkörper (1) getragen wird, eingerichtet sind, wobei die Gaseinlassöffnung (3) das röhrenförmige Element (20) durchquert.

4. Beatmungsmaske nach Anspruch 3, **dadurch gekennzeichnet, dass** das röhrenförmige Element (20) aus der äußeren Oberfläche des Maskenkörpers (1) nach außen vorsteht.

5. Beatmungsmaske nach einem der vorhergehenden Ansprüche, **dadurch gekennzeichnet, dass** das röhrenförmige Element (20) mit dem Maskenkörper (1) integral gemacht ist.

6. Beatmungsmaske nach einem der Ansprüche 4 und 5, **dadurch gekennzeichnet, dass** das röhrenförmige Element (20) mindestens einen Schnitt umfasst, der in dem äußeren umfänglichen Rand (23) des röhrenförmigen Elements (20) gemacht ist, um die mindestens eine Kerbe (21) zu bilden.

7. Beatmungsmaske nach einem der Ansprüche 4 und 5, **dadurch gekennzeichnet, dass** das röhrenförmige Element (20) eine innere zylindrische Oberfläche hat, die die Innenlippe (22) trägt.

8. Beatmungsmaske nach einem der vorhergehenden Ansprüche, **dadurch gekennzeichnet, dass** mehrere Lippen (31) von langen und kurzen Fingern (30, 32) getragen werden, die auf dem hohlen Steckverbinder (4) eingerichtet sind, wobei die kurzen Finger (32) eine Länge haben, die kürzer ist als die der langen Finger (30).

9. Beatmungsmaske nach einem der vorhergehenden Ansprüche, **dadurch gekennzeichnet, dass** die langen und kurzen Finger (30, 32), die auf dem hohlen Steckverbinder (4) eingerichtet sind, nach außen in Bezug zu der umfänglichen Kante (34) des proximalen Endes (4a) des Steckverbinders (4) vorstehen, wobei jeder der langen und kurzen Finger (30, 32) an seinem distalen Ende an dem proximalen Ende (4a) des hohlen Steckverbinders (4) befestigt ist und ferner eine Außenlippe (31) auf seiner äußeren Oberfläche trägt, die an seinem distalen Ende einen kleinen Anschlag bildet, der radial zu dem Äußeren des hohlen Steckverbinders (4) ausgerichtet ist.

10. Beatmungsmaske nach einem der vorhergehenden Ansprüche, **dadurch gekennzeichnet, dass** der Steckverbinder (4) zwischen 2 und 20 lange und kurze Finger (30, 32) umfasst, wobei die Länge der kurzen Finger (32) 3 bis 6 mm beträgt, und die Länge der langen Finger (30) 6 bis 12 mm beträgt.

11. Beatmungsmaske nach einem der vorhergehenden Ansprüche, **dadurch gekennzeichnet, dass**, wenn das proximale Ende (4a) des hohlen Steckverbinders (4) in das röhrenförmige Element (20) eingefügt wird, der Steckverbinder (4) zurückgehalten wird:
- in der ersten axialen Position, von den Außenlippen (31) der langen Finger (30), die gegen die Innenlippe (22) des röhrenförmigen Elements (20) zum Anschlagen kommen, und
- in der zweiten axialen Position von den Außenlippen (31) der kurzen Finger (32), die mit der Innenlippe (22) zum Anschlagen kommen, verriegelt.

12. Beatmungsmaske nach einem der vorhergehenden Ansprüche, **dadurch gekennzeichnet, dass** der Anschlag (35) derart bemessen ist, dass er eine Form und eine Größe hat, die zu der Form der mindestens einen Kerbe (21) des röhrenförmigen Elements (20) passen.

13. Beatmungsmaske nach einem der vorhergehenden Ansprüche, **dadurch gekennzeichnet, dass** die Beatmungsmaske eine Gesichtsmaske ist.

14. Beatmungsmaske nach einem der vorhergehenden Ansprüche, **dadurch gekennzeichnet, dass** die Innenkammer (2) einen umfänglichen Rand (11) und ein Kissen (12), das an dem umfänglichen Rand (11) befestigt ist, umfasst, wobei das Kissen (12) eine zentrale Öffnung zum Aufnehmen mindestens eines Teils der Nase und/oder des Munds des Patienten hat.

15. Beatmungsmaske nach einem der vorhergehenden Ansprüche, **dadurch gekennzeichnet, dass** die Beatmungsmaske ferner eine Stirnstütze (9), ein Kopfgeschirr und Befestigungsmittel (19, 20) zum Befestigen des Kopfgeschirrs an dem Maskenkörper (1) umfasst.

## Revendications

1. Masque respiratoire comprenant un corps de masque (1) avec une chambre interne (2) et un orifice d'admission de gaz (3), ledit orifice d'admission de gaz (3) ayant un axe (AA), ledit orifice d'admission de gaz (3) étant en communication fluidique avec ladite chambre interne (2), et un raccord creux (4) avec un passage de gaz interne (5) étant raccordé audit orifice d'admission de gaz (3) de sorte que le passage interne (5) du raccord creux (4) soit en communication fluidique avec ladite chambre interne (2), ledit raccord creux (4) pouvant tourner autour de l'axe (AA), dans lequel :
- le raccord creux (4) est en outre mobile en translation le long de l'axe (AA) entre au moins : une première position axiale, dans lequel le raccord peut tourner librement autour dudit axe (AA) et une seconde position axiale, dans lequel le raccord est verrouillé dans une position fixe et ne peut pas tourner librement autour dudit axe (AA),
- et le masque comprend en outre des moyens de verrouillage pour verrouiller et pour maintenir le raccord creux (4) bloqué dans ladite seconde position axiale, lesdits moyens de verrouillage comprenant des lèvres (22, 31) comportant une lèvre interne (22) supportée par le corps de masque (1) et des lèvres internes (31) supportées par le raccord creux (4),
**caractérisé en ce que** les moyens de verrouillage comprennent en outre au moins une entaille (21) supportée par le corps de masque (1), et au moins une butée (35) supportée par le raccord creux (4), ladite au moins une butée (35) pénétrant au moins partiellement à l'intérieur de ladite entaille (21) pour bloquer la rotation du raccord creux autour de l'axe (AA), lorsque le raccord creux (4) est dans la seconde position axiale.

2. Masque respiratoire selon la revendication 1, **caractérisé en ce que** le raccord creux (4) a une forme coudée.

3. Masque respiratoire selon l'une quelconque des revendications précédentes, **caractérisé en ce que** la lèvre interne (22) et l'au moins une entaille (21) sont agencées sur un élément tubulaire (20) supporté par le corps de masque (1), l'orifice d'admission de gaz (3) traversant ledit élément tubulaire (20).

4. Masque respiratoire selon la revendication 3, **caractérisé en ce que** l'élément tubulaire (20) fait saillie vers l'extérieur de la surface externe du corps de masque (1).

5. Masque respiratoire selon l'une quelconque des revendications précédentes, **caractérisé en ce que** l'élément tubulaire (20) est fait d'un seul tenant avec le corps de masque (1).

6. Masque respiratoire selon l'une quelconque des revendications 4 et 5, **caractérisé en ce que** l'élément tubulaire (20) comprend au moins une découpe faite dans la bordure périphérique externe (23) dudit élément tubulaire (20) de sorte à former ladite au moins une entaille (21).

7. Masque respiratoire selon l'une quelconque des revendications 4 et 5, **caractérisé en ce que** l'élément tubulaire (20) a une surface cylindrique interne supportant la lèvre interne (22).

8. Masque respiratoire selon l'une quelconque des revendications précédentes, **caractérisé en ce que** plusieurs lèvres (31) sont supportées par des doigts longs et courts (30, 32) agencés sur le raccord creux (4), les doigts courts (32) ayant une longueur qui est plus courte que celle des doigts longs (30).

9. Masque respiratoire selon l'une quelconque des revendications précédentes, **caractérisé en ce que** les doigts longs et courts (30, 32) agencés sur le raccord creux (4) font saillie vers l'extérieur par rapport au bord périphérique (34) de l'extrémité proximale (4a) du raccord (4), chacun desdits doigts longs et courts (30, 32) étant fixé par son extrémité distale à l'extrémité proximale (4a) du raccord creux (4) et supporte en outre une lèvre externe (31) sur sa surface externe formant, au niveau de son extrémité distale, une petite butée orientée radialement vers l'extérieur du raccord creux (4).

10. Masque respiratoire selon l'une quelconque des revendications précédentes, **caractérisé en ce que** le raccord (4) comprend entre 2 et 20 doigts longs et courts (30, 32), la longueur des doigts courts (32) allant de 3 à 6 mm et la longueur des doigts longs (30) allant de 6 à 12 mm.

11. Masque respiratoire selon l'une quelconque des revendications précédentes, **caractérisé en ce que**, lorsque l'extrémité proximale (4a) du raccord creux (4) est insérée à l'intérieur de l'élément tubulaire (20), ledit raccord (4) est retenu :
- dans la première position axiale, par les lèvres externes (31) des doigts longs (30) qui viennent en butée contre la lèvre interne (22) de l'élément tubulaire (20), et
- verrouillé dans la seconde position axiale, par les lèvres externes (31) des doigts courts (32) venant en butée avec ladite lèvre interne (22).

12. Masque respiratoire selon l'une quelconque des revendications précédentes, **caractérisé en ce que** la butée (35) est dimensionnée de sorte à avoir une forme et taille qui correspondent à la forme de l'au moins une entaille (21) de l'élément tubulaire (20).

13. Masque respiratoire selon l'une quelconque des revendications précédentes, **caractérisé en ce que** le masque respiratoire est un masque facial.

14. Masque respiratoire selon l'une quelconque des revendications précédentes, **caractérisé en ce que** la chambre interne (2) comprend une bordure périphérique (11) et un coussin (12) étant fixé à ladite bordure périphérique (11), ledit coussin (12) ayant une ouverture centrale pour recevoir au moins une partie du nez et/ou de la bouche du patient.

15. Masque respiratoire selon l'une quelconque des revendications précédentes, **caractérisé en ce que** le masque respiratoire comprend en outre un appui frontal (9), un harnais crânien et des moyens de fixation (19, 20) pour fixer le harnais crânien au corps de masque (1).
